# EUROPEAN PATENT APPLICATION

(11) **EP 3 189 783 A1**
(43) Date of publication of application: **12.07.2017**
(21) Application number: 14901359.1
(22) Date of filing: 01.09.2014
(51) Int. Cl.: A61B 5/15, A61M 5/00

(54) **LIGHTING DEVICE**

(71) Applicant: Synqroa Co. Ltd., Tokyo 108-0073 (JP)
(72) Inventor: KOYAMA, Mitsuhiro, Tokyo 108-0073 (JP); AYABE, Kaori, Tokyo 108-0073 (JP); SHIMADA, Jun, Tokyo 108-0073 (JP)
(74) Representative: Detken, Andreas
(86) International application number: PCT/JP2014/004475
(87) International publication number: WO 2016/035106

(57) **Abstract**

By providing a surface light source that planarly emits first illuminating light FL in the blue wavelength region of 400 to 500 nm and second illuminating light SL in the red wavelength region of 600 to 660 nm, and by emitting illuminating light in these specific wavelength region, it makes it possible to visually identify the position of veins easily.

## Description

### Technical Field

The present invention relates to a lighting device that can visually identify the position of veins easily when applying, for example, a treatment such as intravenous injection, blood sampling or the like to patients.

### Background Art

Conventionally, in medical sites, when applying a treatment such as intravenous injection or blood sampling to a patient, by wrapping a tourniquet at a position nearer to the body trunk than a part to be punctured to cause the venous blood to be congested, blood vessels (veins) comes up to the skin surface, thereby grasping the position of the blood vessels of the patient.

Further, Patent Document 1 proposes a vein investigation device that can confirm the position of the veins based on difference in amount of reflected light caused by difference in absorption characteristics between veins and other tissues. Patent Document 2 discloses a belt-type translucent illuminator for blood sampling in which light is transmitted from the both side surfaces and the back surface of the arms of a patient, whereby shadows of veins can be seen at the inside of the elbow.

### Related Art Documents

### Patent Documents

Patent Document 1: JP-A-H02-172473
Patent Document 2: Registered Utility Model No. 3144999

### Summary of the Invention

### Problems to be Solved by the Invention

In Patent Document 1, by irradiating light having a wavelength in the red to infrared region (700 to 2000 nm), veins are probed by matching the positions of two cursors based on difference in amount of reflected light between veins and other tissues. However, in this technology, visual identification of the position of veins has not been attained yet. The technology in this document only assumes that veins are present below a line that connects two cursors for which positioning has been conducted.

The invention of Patent Document 2 requires not only a troublesome operation of wrapping a belt around a patient's arms or the like is required, but also a cooling mechanism for avoiding burns when the light-emitting unit is in close contact with the skin of the patient.

The present invention has been made taking the above-mentioned circumstances into consideration, and is aimed at providing a lighting device capable of visually identifying the position of veins easily only by irradiating a part for which the position of veins is required to be identified with illuminating light without the need of a troublesome operation of wrapping a belt, etc.

### Means for Solving the Problems

The lighting device according to the present invention is a lighting device for visually identifying the position of veins, comprising a light source that emits first illuminating light in the blue wavelength region of 400 to 500 nm and second illuminating light in the red wavelength region of 600 to 660 nm.

### Advantageous Effects of the Invention

According to the invention, it is possible to identify visually the position of veins easily only by irradiating a part for which the position of veins is required to be identified with illuminating light in a specific wavelength region.

### Brief Description of the Drawings

Fig. 1 is one example of an ideal spectral distribution of light emitted from a light source provided in the lighting device according to the embodiment of the present invention;
Fig. 2 is an explanatory view showing Example 1 of the lighting device according to the embodiment of the present invention;
Fig. 3 is an explanatory view showing Example 2 of the lighting device according to the embodiment of the present invention;
Fig. 4 is an explanatory view showing Example 3 of the lighting device according to the embodiment of the present invention; and
Fig. 5 (a) is a photograph showing how veins are seen when the lighting device according to the present invention is used, and Fig. 5(b) is a photograph showing how veins are seen when a fluorescent lamp is used.

### Mode for Carrying out the Invention

Hereinbelow, the embodiment of the lighting device according to the present invention will be explained.

The lighting device of the present embodiment is used for visually identifying the position of veins to be punctured when applying, for example, a treatment such as intravenous injection or blood sampling to a patient. The lighting device is provided with a light source that emits first illuminating light in the blue wavelength region and second illuminating light in the red wavelength region, and is configured to emit the light in these specific wavelength regions to a part to be punctured of a patient's arm or the like.

Oxidized hemoglobin in which hemoglobin contained in red blood cells in the blood is bound with oxygen absorbs little light in the red wavelength region. For this reason, while arterial blood containing a large amount of oxidized hemoglobin appears red, venous blood containing a large amount of oxygen-free reduced hemoglobin appears bluish black. The reason therefor is that, the amount of absorbed light in the blue wavelength region of the reduced hemoglobin, is greatly reduced as compared with the oxidized hemoglobin. In the present embodiment, by utilizing such light absorption characteristics of the reduced hemoglobin, visual identification of the position of veins becomes possible.

That is, for the above-mentioned light absorption characteristics, the reduced hemoglobin that is contained in a large amount in the venous blood hardly absorbs the first illuminating light in the blue wavelength region and the second illuminating light in the red wavelength region that are emitted from the surface light source of the lighting device of the present embodiment. For this reason, part of the first illuminating light in the blue wavelength region and part the second illuminating light in the red wavelength region, which are emitted to a part for which the position of veins is desired to be identified, are reflected by the reduced hemoglobin which is contained in a large amount in the venous blood, and the reflected light allows veins to be seen through the skin surface of the patient. As a result, the position of veins to be punctured can be visually identified.

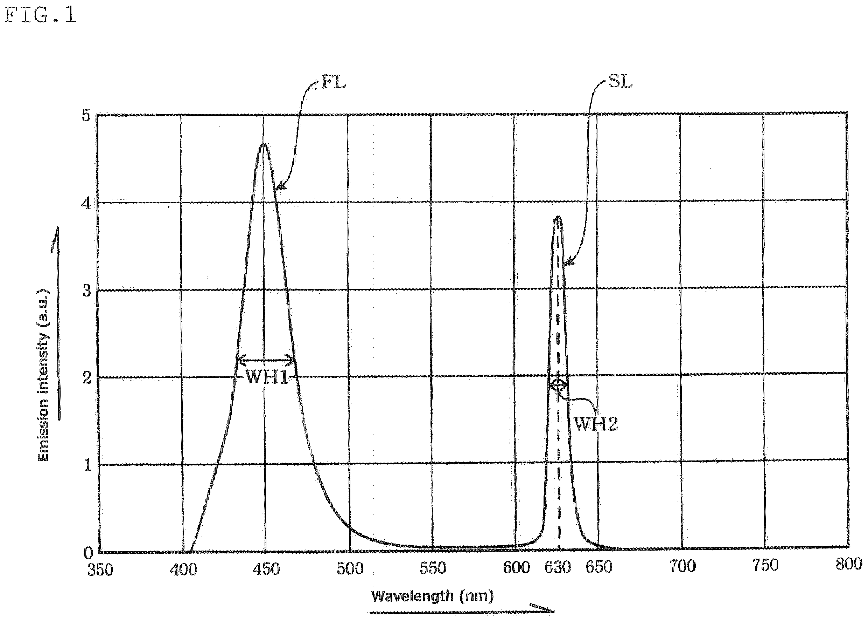

Based on such a principle, when visually identifying the position of veins to be punctured, the first illuminating light FL in the blue wavelength region is set to have a wavelength region of 400 to 500 nm which is hardly absorbed by the reduced hemoglobin and the second illuminating light SL in the red wavelength region is set to have a wavelength region of 600 to 660 nm which is hardly absorbed by the reduced hemoglobin. In order to allow most of illuminating light emitted from a light source provided in the lighting device of the present embodiment to be reflected by the reduced hemoglobin, it is preferred that the first illuminating light FL contain spectral components that have a maximum peak in the wavelength region of 440 to 460 nm in the blue wavelength region of 400 to 500 nm and have a full width at half maximum (WH1) of 100 nm or less (preferably 70 nm or less) and the second illuminating light SL contain spectral components that have a maximum peak in the wavelength region of 620 to 640 nm in the red wavelength region of 600 to 660 nm and have a full width at half maximum (WH2) of 60 nm or less, preferably 25 nm or less (see Fig. 1).

Fig. 1 is an example of an ideal spectral distribution of illuminating light emitted from a light source provided in the lighting device of the present embodiment. The spectral distribution of the illuminating light may be such that each of the first illuminating light FL and the second illuminating light SL contains at least the spectral component mentioned above. If the spectral components are contained, most of them are reflected by the reduced hemoglobin and identification of veins becomes easier. Within an amount range that does not interfere the identification of veins, the first illuminating light FL and the second illuminating light SL may contain other components than the above-mentioned spectral components.

Further, within an amount range that does not interfere identification of veins, the illuminating light emitted from the light source provided in the lighting device of the present embodiment may contain light of a wavelength region other than the wavelength region of 400 to 500 nm (first illuminating light) and the wavelength region of 600 to 660 nm (second illuminating light).

In order to allow veins to be seen more clearly on the skin surface of a patient by illuminating light emitted from the lighting device of the present embodiment, it is preferable to adjust such that the amount of the first illuminating light FL in the blue wavelength region becomes larger than the amount of the second illuminating light SL in the red wavelength region. More specifically, the ratio of the amount of the first illuminating light FL in the blue wavelength region and the amount of the second illuminating light SL in the red wavelength region is preferably 10:8 to 10:6.

The amount of light is obtained from an integral value of the emission intensity [a.u. (arbitrary unit)] in each wavelength region.

The reduced hemoglobin has absorption characteristics absorbing the light in the green wavelength region. Therefore, the light in the green wavelength region does not contribute to specifying the position of veins at all. Moreover, if the light in the, green wavelength region is mixed with the first illuminating light FL in the blue wavelength region reflected by veins and the second illuminating light SL in the red wavelength region, the color of light becomes white, whereby identification of veins is interfered.

For the above-mentioned reason, it is preferred that the illuminating light emitted from the light source provided in the lighting device of the present embodiment contain light in the green wavelength region in a small amount as possible. More specifically, the amount of light in the green wavelength region of 540 to 580 nm emitted from a surface light source is preferably 5% or less of the amount of the first illuminating light FL. It is more preferable to allow the amount of light in the green wavelength region be substantially zero by means of an optical filter such as a cut filter.

In addition, in order to make it possible to visually identify the position of veins, when the illuminating light is emitted to a part for which the position of veins is desired to be identified, in order to clearly distinguish the light reflected by veins from the light reflected by other parts than veins, it is desired that the skin surface be uniformly irradiated with illuminating light and that light be uniformly reflected by the tissue beneath the skin. The specific configuration of the light source provided in the lighting device of the present embodiment is not specifically restricted. For the above-mentioned reason, the light source provided in the lighting device of the present embodiment may be a surface light source that planarly emits the first illuminating light FL in the blue wavelength region and the second illuminating light SL in the second wavelength region.

As the light source provided in the lighting device of the present embodiment, one that emits illuminating light in a specific wavelength region by using an incandescent lamp, a discharge lamp, a laser, a light-emitting diode or the like as a primary light source and by using an optical filter that transmits only light having a specific wavelength such as a band pass filter, a cut filter, or the like can be used. If the light source is a surface light source, the light source may be configured to emit illuminating light planarly by a diffusion plate, a light guide plate or the like.

In the present embodiment that requires illuminating light in a specific wavelength region, it is preferable to use a light-emitting diode (LED) that can control the wavelength of emitted light by preparing a light-emitting material. Among light-emitting diodes, an organic electroluminescence (organic EL) is particularly preferable since diffused light with no unevenness due to surface emission can be obtained even if it is used singly, and optical distribution property that shows how light is spread is also excellent. In addition to emitting natural soft light without dazzling due to surface emission, an organic EL also has an advantage that shadow cast by the hand of a practitioner who conducts puncture hardly occurs due to an ideal spherical orientation.

As mentioned above, according to the lighting device according to the present embodiment, the position of veins can be visually identified only by irradiating a part for which the position of veins is desired to be identified with illuminating light, and as a result, a troublesome operation of winding a belt around a patient's arm, etc. that is conducted in the technology of Patent Document 2 is not necessary. Therefore, in the case where a patient who undergoes a treatment such as intravenous injection or blood sampling is an infant, in particular, an infant who requires care in an incubator after the birth due to unhealthy growth, the position of veins can be identified easily for a short period of time, whereby an accident caused by wrong puncture can be prevented beforehand.

### EXAMPLES

Subsequently, the specific example of the lighting device according to the present embodiment will be explained with reference to the drawings.

### [Example 1]

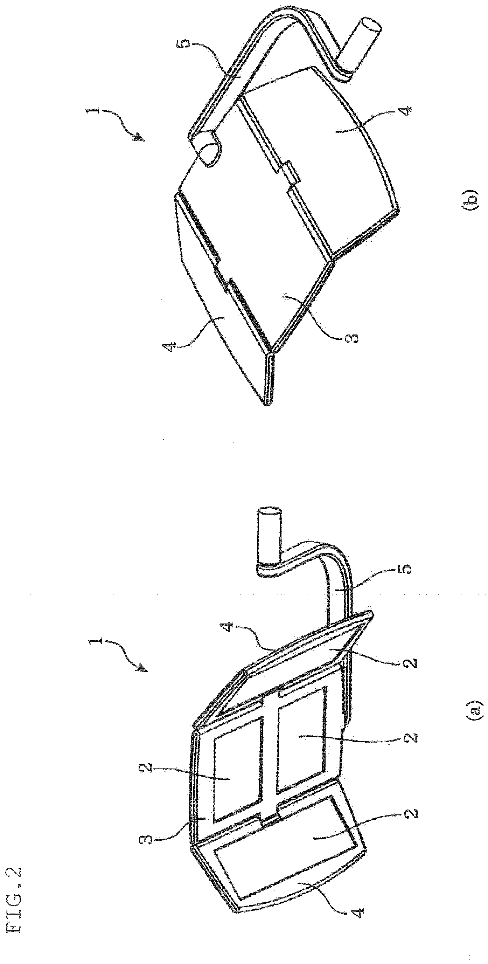

A lighting device 1 shown in Fig. 2 is provided with an illuminating unit 3 attached to an arm 5 and a sub-illuminating unit 4 openably/closably attached to the both side edges of the illuminating unit 3. Each of the illuminating unit 3 and the sub-illuminating unit 4 is provided with a surface light source 2 that planarly emits the first illuminating light and the second illuminating light in the above-mentioned wavelength region. As examples of such surface light source 2, a LED in which a diffusion plate is used or an organic EL can be used.

Fig. 2(a) is a front perspective view of this example, and Fig. 2(b) is a rear perspective view of this example.

The lighting device 1 supported by the arm 5 may be of a stand type lightening device with the arm 5 being attached to a stand or may be of a fixed type lighting device by attaching the arm 5 to a ceiling, a wall, a bed, a chair or the like. In the example shown in Fig. 2, the arm 4 has a turning part, whereby it is possible to perform positioning when irradiating a part for which position of veins is desired to be identified with light. It is also possible to enhance the degree of freedom in positioning by using it as a flexible arm.

In such lighting device 1, the arm 5 is adjusted so that a part for which the position of veins is desired to be identified is irradiated with illuminating light emitted from the illuminating unit 3 and the sub-illuminating unit 4, and at the same time, the opening/closing angle of the sub-illuminating part 4 is appropriately adjusted, whereby the position is irradiated with illuminating light. As a result, veins are allowed to be seen through the skin surface of the patient, whereby a part of veins to be punctured can be visually identified easily.

### [Example 2]

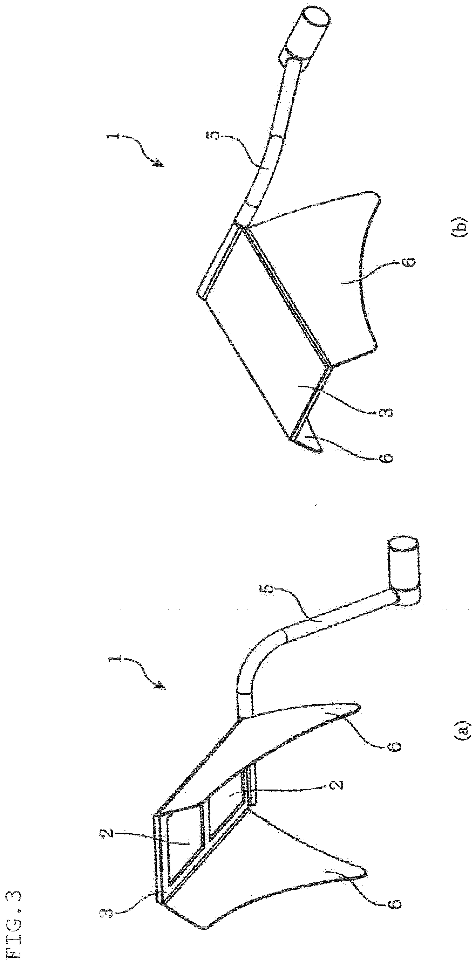

The lighting device 1 shown in Fig. 3 is provided with the illuminating unit 3 attached to the arm 5 and a light-shielding board 6 openably/closably attached to the both side edges of the illuminating unit 3. It has almost the same configuration as that shown in Fig. 2, except that the light-shielding board 6 is provided instead of the secondary illuminating part 4.

Fig. 3(a) is a front perspective view of the present embodiment, and Fig. 3(b) is a rear perspective view of the present embodiment.

### [Example 3]

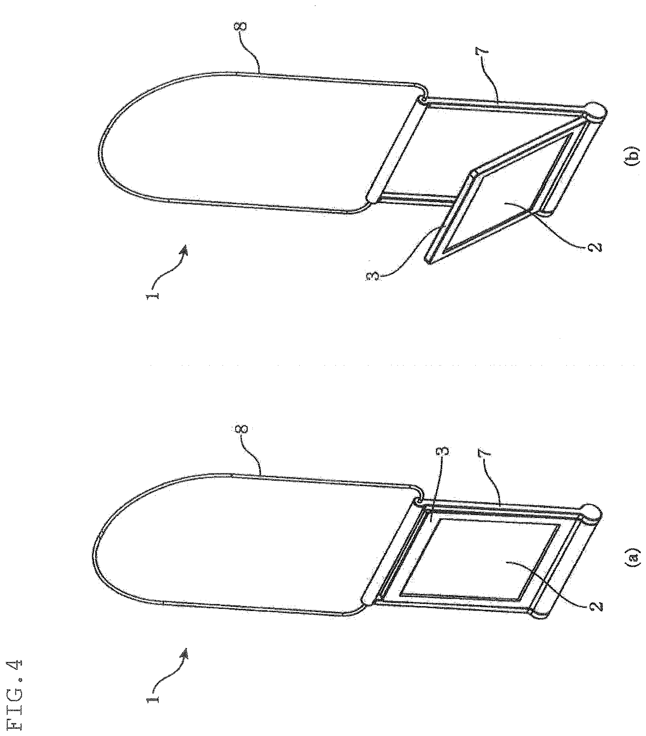

The lighting device 1 shown in Fig. 4 is provided with the illuminating unit 3 to which one of frames 7 is turnably attached as a turning shaft, and the device is hung on the neck of a practitioner by a wire 8 attached to the frame 7.

Such a lighting device 1 is preferably used by being carried by a practitioner who makes round visits to the beds of his/or her patients in hospital rooms to perform a treatment such as intravenous injection and blood sampling or the like. When using the device, the illuminating unit 3 is rotated such that a part for which the position of veins is desired to be identified is irradiated with illuminating light emitted from the illuminating unit 3, and the part is irradiated with illuminating light emitted from the surface light source 2. As a result, veins are allowed to be seen through the skin surface of the patient, whereby the position of veins to be punctured can be visually identified easily.

Fig. 4(a) is a front perspective view showing a state before the illuminating unit 3 is rotated in the present example, and Fig. 4(b) is a rear perspective view showing a state after the illuminating unit 3 is rotated in the present example.

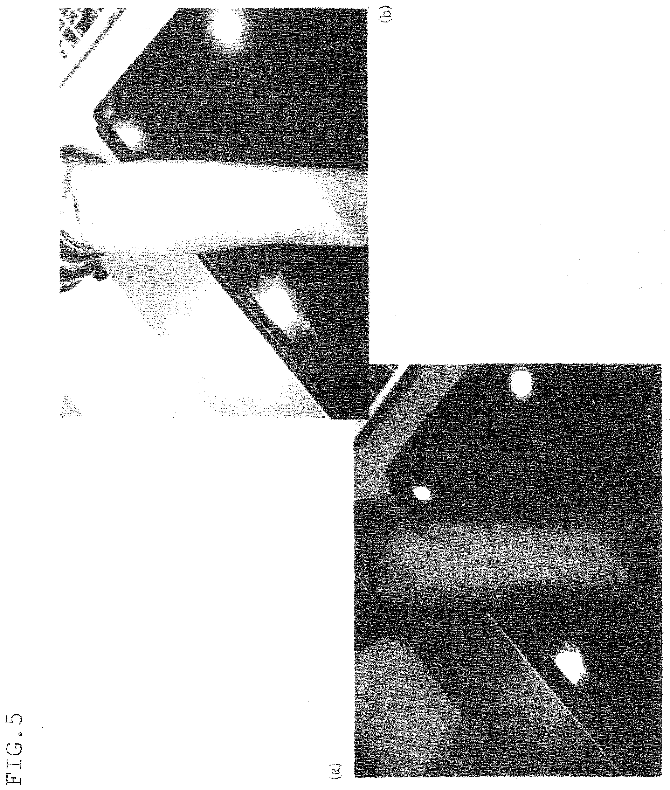

Here, Fig. 5(a) is an example showing how veins are seen when a part of a patient for which the position of veins is desired to be identified is irradiated planarly with the first illuminating light with the blue wavelength region of 400 to 500 nm and the second illuminating light with the red wavelength region of 600 to 660 nm at a light amount ratio of 10:8. It can be confirmed that veins are seen through the skin surface of the patient.

Meanwhile, the first illuminating light has a maximum peak at a wavelength of 450 nm in the blue wavelength region of 400 to 500 nm, and contains spectral components having a full width at half maximum of about 68 nm, and the second illuminating light has a maximum peak at a wavelength of 630 nm in the red wavelength region of 600 to 660 nm and contains spectral components having a full width at half maximum of about 21 nm.

Fig. 5(b) is an example showing how veins are seen when the arm of the same patient is irradiated with illuminating light emitted from a fluorescent lamp. It can be confirmed that veins are not seen clearly.

Hereinabove, the present invention is explained with reference to preferred embodiments, but the present invention is not restricted to the above-mentioned embodiment. It is needless to say that various modifications are possible within the scope of the present invention.

In the above-mentioned embodiment, an example is given in which the position of veins to be punctured is visually identified when a treatment such as intravenous injection and blood sampling or the like is conducted for a patient. The present invention is applied not only to intravenous injection or blood sampling but also to puncture into veins at the time of dialysis. In addition, the present invention may be applied when a surgery is conducted; specifically, when incised parts are treated, the position of veins can be identified visually by a practitioner so that the veins are prevented from being injured at the time of a surgery.

### Industrial Applicability

As explained above, the present invention can be utilized in various medical fields since veins can be identified easily.

### Description of Symbols

1. Lighting device
2. Surface light source
3. Illuminating unit
4. Sub-illuminating unit
5. Arm
6. Light-shielding plate
7. Frame
8. Wire

## Claims

1. A lighting device for visually identifying the position of veins, that comprises a light source that emits first illuminating light in the blue wavelength region of 400 to 500 nm and second illuminating light in the red wavelength region of 600 to 660 nm.

2. The lighting device according to claim 1, wherein the amount of light in the green wavelength region of 540 to 580 nm in illuminating light emitted from the surface light source is 5% or less of the amount of the first illuminating light.

3. The lighting device according to claim 1 or 2, that is provided with a surface light source that planarly emits the first illuminating light and the second illuminating light.

4. The lighting device according to any one of claims 1 to 3, wherein the amount ratio of the first illuminating light and the second illuminating light is 10:8 to 10:6.
